# EUROPEAN PATENT APPLICATION

(11) **EP 3 489 193 A1**
(43) Date of publication of application: **29.05.2019**
(21) Application number: 18208702.3
(22) Date of filing: 27.11.2018
(51) Int. Cl.: C01B 21/086, C01B 21/093, H01M 10/052

(54) **SULFONYLIMIDE COMPOUND, PRODUCTION METHOD THEREOF, ELECTROLYTE COMPOSITION, ELECTROLYTIC SOLUTION AND LITHIUM ION BATTERY**

(30) Priority: 28.11.2017 JP 2017228257; 28.11.2017 JP 2017228271
(71) Applicant: Nippon Shokubai Co., Ltd., Osaka-shi, Osaka 541-0043 (JP); Morita Chemical Industries Co., Ltd., Osaka-shi, Osaka 541-0056 (JP)
(72) Inventor: MORITA, Yoshihiro, Suita-shi,, Osaka 564-8512 (JP); MIZUNO, Hiroyuki, Suita-shi,, Osaka 564-8512 (JP); FUKATA, Yukihiro, Suita-shi,, Osaka 564-8512 (JP); SHIBATA, Shin-ya, Suita-shi,, Osaka 564-8512 (JP); ITAYAMA, Naohiko, Suita-shi,, Osaka 564-8512 (JP); KATSUYAMA, Hiromoto, Suita-shi,, Osaka 564-8512 (JP)
(74) Representative: Enomoto, Kéi

(57) **Abstract**

A compound represented by the following formula (1): 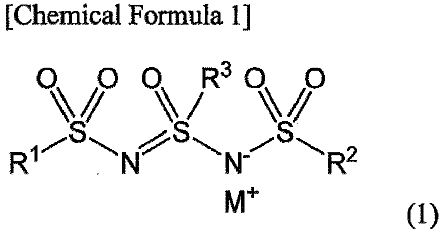 wherein R¹, R² and R³ each independently represent fluorine, an alkyl group having 1 to 6 carbon atoms or a fluoroalkyl group having 1 to 6 carbon atoms; and M⁺ represents an alkali metal ion.

## Description

### TECHNICAL FIELD

The present invention relates to a sulfonylimide compound, a production method thereof, an electrolyte composition, an electrolytic solution and a lithium ion battery.

### BACKGROUND

A fluorosulfonylimide salt or a derivative thereof is useful as an intermediate of a compound having an N(SO₂F) group or an N(SO₂F)₂ group. The fluorosulfonylimide salt or a derivative thereof is also used as, e.g., an electrolyte, an additive in an electrolytic solution of a battery cell or a capacitor, a selective electrophilic fluorinating agent, a photoacid generator, a thermal acid generator and a near-infrared absorbing dye and thus useful as various use applications (Patent literature 1).

### Citation List

### Patent Literature

Patent Literature 1: International Publication No. WO2011/149095

### SUMMARY

However, in the above use applications, a compound may be sometimes used under high temperature environments. Because of this, it is desired to develop a compound enhanced in heat resistance. A salt or derivative of fluorosulfonylimide is useful as, e.g., an electrolyte, as mentioned above; however, there is a room for improvement in view of heat resistance. Because of this, it is required to develop a novel compound more excellent in heat resistance.

The present invention was achieved in view of the aforementioned circumstances and is directed to providing a novel compound excellent in heat resistance and an electrolyte composition using the compound.

The sulfonylimide compound of the present invention is represented by the following formula (1): 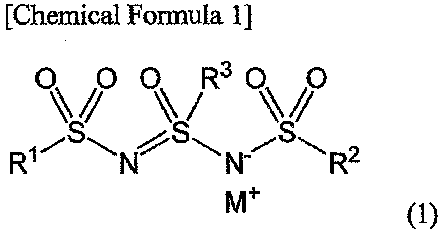 wherein R¹, R² and R³ each independently represent fluorine, an alkyl group having 1 to 6 carbon atoms or a fluoroalkyl group having 1 to 6 carbon atoms; and M⁺ represents an alkali metal ion.

In the formula (1), it is preferable that each R¹, R² and R³ is a fluorine atom, and M⁺ is Li⁺.

The electrolyte composition of the present invention comprises a compound represented by the following formula (1) and a compound represented by the following formula (2), in which the content of the compound of the formula (1) in the electrolyte composition is 1 mass ppm to 200000 mass ppm; and the content of the compound of the formula (2) is 100000 mass ppm or more, 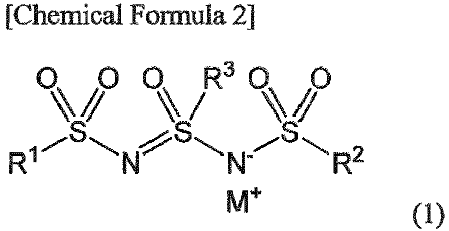 wherein R¹, R² and R³ each independently represent fluorine, an alkyl group having 1 to 6 carbon atoms or a fluoroalkyl group having 1 to 6 carbon atoms; and M⁺ represents an alkali metal ion,

(XSO₂)(XSO₂)NM ... (2)

wherein X represents a fluorine atom, an alkyl group having 1 to 6 carbon atoms or a fluoroalkyl group having 1 to 6 carbon atoms; a plurality of X may be the same or different; and M represents an alkali metal.

It is preferable that, in the above electrolyte composition, each R¹, R² and R³ in the formula (1) is a fluorine atom; M⁺ is Li⁺; and each X in the formula (2) is a fluorine atom.

It is preferable that the above electrolyte composition further comprises 5 mass ppm to 100000 mass ppm of MFSO₃ (M represents an alkali metal).

The production method of the present invention is a method for producing a compound of the following formula (1), comprising a step of heating a solid-state compound of the following formula (2) at 150°C or more, 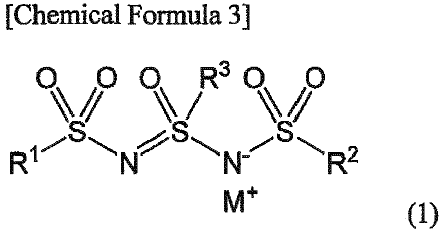 wherein R¹, R² and R³ each independently represent fluorine, an alkyl group having 1 to 6 carbon atoms or a fluoroalkyl group having 1 to 6 carbon atoms; and M⁺ represents an alkali metal ion,

(XSO₂)(XSO₂)NM ... (2)

wherein X represents a fluorine atom, an alkyl group having 1 to 6 carbon atoms or a fluoroalkyl group having 1 to 6 carbon atoms; a plurality of X may be the same or different; and M represents an alkali metal.

The solution of the present invention comprises 0.1 mass ppm to 180000 mass ppm of a compound represented by the following formula (4): 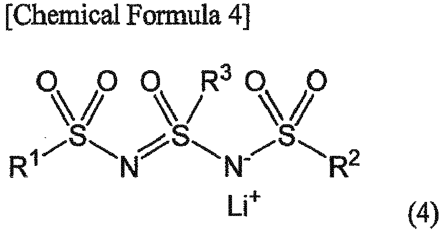 wherein R¹, R², R³ each independently represent fluorine or a fluoroalkyl group having 1 to 6 carbon atoms.

It is preferable that the solution of the present invention further comprises 0.1 mass ppm to 10000 mass ppm of LiFSO₃.

It is preferable that the solution of the present invention further comprises at least one compound selected from the group consisting of LiPF₆, LiBF₄ and LiN(SO₂CₙF₂ₙ₊₁)₂ wherein n = an integer of 0 to 6.

It is preferable that the solution of the present invention further comprises 1 mass ppm to 90 mass% of lithium bis(fluorosulfonyl)imide.

It is preferable that, in the formula (4), each R¹, R² and R³ is a fluorine atom.

The electrolytic solution of the present invention comprises the above solution.

The lithium ion battery of the present invention comprises the above electrolytic solution.

According to the present invention, it is possible to provide a compound having high heat resistance and an electrolyte composition comprising the compound. According to the present invention, it is also possible to provide a solution suitable for use in a high temperature condition, an electrolytic solution excellent in high-temperature properties and a lithium ion battery using the electrolytic solution.

### DETAILED DESCRIPTION

Now, an embodiment of the present invention will be more specifically described.

The sulfonylimide compound of the embodiment is represented by the following formula (1): 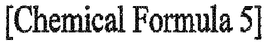 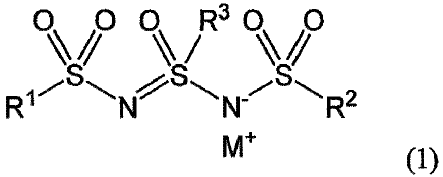 wherein R¹, R² and R³ each independently represent fluorine, an alkyl group having 1 to 6 carbon atoms or a fluoroalkyl group having 1 to 6 carbon atoms; and M⁺ represents an alkali metal ion.

The alkyl group having 1 to 6 carbon atoms is used as R¹, R² and R³ may be linear or branched. The fluoroalkyl group having 1 to 6 carbon atoms may be linear or branched. Note that, the fluoroalkyl group having 1 to 6 carbon atoms may be the one in which the hydrogen atoms of an alkyl group having 1 to 6 carbon atoms are substituted with one or more fluorine atoms and may be a perfluoroalkyl group in which all hydrogen atoms are substituted with fluorine atoms.

In the above formula (1), M⁺ represents an alkali metal ion. Examples of the alkali metal ion include Li⁺, Na⁺, K⁺, Rb⁺ and Cs⁺. Of them, at least one selected from the group consisting of Li⁺, Na⁺ and K⁺ is preferable and Li⁺ is more preferable.

In the formula (1), R¹, R² and R³ may be different or the same. It is preferable that each R¹, R² and R³ is a fluorine atom.

The compound in which each R¹, R² and R³ is a fluorine atom is an alkali metal salt of N,N-bis(fluorosulfonyl)imidosulfamoyl fluoride represented by the following formula (3): 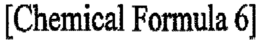 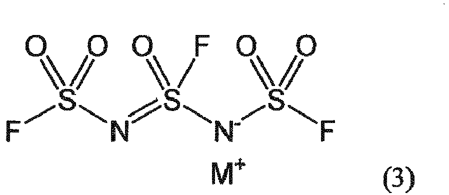 wherein M⁺ represents an alkali metal ion.

In the above formula (3), M⁺ represents an alkali metal ion. Examples of the alkali metal ion include Li⁺, Na⁺, K⁺, Rb⁺ and Cs⁺. Of them, at least one selected from the group consisting of Li⁺, Na⁺ and K⁺ is preferable and Li⁺ is more preferable.

The compound of the above formula (3) is further excellent in heat resistance and thus particularly usefully applied to uses requiring heat resistance, such as an electrolytic solution for, e.g., a battery cell or a capacitor.

The compound of the above formula (1) may be used alone or in the form of an electrolyte composition containing the compound of the following formula (1) and other components. The electrolyte composition is a solid-state electrolyte composition and may be a powder mixture. In the powder mixture, the compound of the above formula (1) may be contained as particles of the compound of the formula (1) or a single particle formed of the compound together with other components.

In an embodiment of the present invention, the electrolyte composition contains a compound represented by the above formula (1) and a compound represented by the following formula (2) and the content of the compound of the formula (1) in the electrolyte composition is 1 mass ppm to 200000 mass ppm and the content of the compound of the formula (2) is 100000 mass ppm or more,

(XSO₂)(XSO₂)NM ... (2)

wherein X represents a fluorine atom, an alkyl group having 1 to 6 carbon atoms or a fluoroalkyl group having 1 to 6 carbon atoms; a plurality of X may be the same or different; and M represents an alkali metal and may be the same alkali metal as M⁺ in the formula (1).

Note that, in the specification, the contents of individual components in the electrolyte composition are proportions (e.g., mass ppm) to the total amount of the electrolyte composition.

The alkyl group having 1 to 6 carbon atoms as X may be linear or branched. The fluoroalkyl group having 1 to 6 carbon atoms may be linear or branched. Note that, the fluoroalkyl group having 1 to 6 carbon atoms may be the one in which the hydrogen atoms of an alkyl group having 1 to 6 carbon atoms are substituted with one or more fluorine atoms and may be a perfluoroalkyl group in which all hydrogen atoms are substituted with fluorine atoms..

The electrolyte composition may contain an electrolyte other than a compound of the formula (1) and a compound of the formula (2). As the electrolyte other than the compound of the formula (1), an alkali metal salt except the compound of the formula (1) is mentioned. Examples of the anion of the alkali metal salt include a hexafluorophosphate, a perchlorate, a tetrafluoroborate, a hexafluoroantimonate, a fluorosulfate, a perfluoroalkyl sulfate (e.g., trifluorosulfate, pentafluoroethyl sulfate), a tris(perfluoroalkylsulfonyl)methide salt (e.g., tris(trifluoromethylsulfonyl)methide salt) and a bisoxalate. Of them, fluorosulfate or a bis(fluorosulfonyl)imide salt is preferable, and bis(fluorosulfonyl)imide salt is more preferable. The alkali metals contained in these electrolytes are not particularly limited; however, at least one selected from the group consisting of Li⁺, Na⁺ and K⁺ is preferable and Li⁺ is more preferable. The alkali metals contained in these alkali metal salts may be the same as or different from the alkali metal ions contained in the compound of the formula (1).

The content of a compound of the above (1) in the electrolyte composition is not particularly limited. In order to improve the heat resistance of the electrolyte composition, the content is preferably 1 mass ppm to 200000 mass ppm, more preferably 100 mass ppm to 15000 mass ppm, and further preferably 4000 mass ppm to 10000 mass ppm. In order to improve high-temperature properties of a battery when the electrolyte composition is used as an electrolytic solution and in order to obtain sufficient ionic conductivity of the electrolytic solution, the content of a compound of the above (1) in the electrolyte composition may be 50000 mass ppm or more, 50000 mass ppm to 200000 mass ppm or 100000 mass ppm to 200000 mass ppm.

The content of a compound of the formula (2) in the electrolyte composition, in order to suppress an increase of internal resistance when the composition is used as an electrolytic solution, is preferably 100000 mass ppm to 999990 mass ppm and more preferably 100000 mass ppm to 995000 mass ppm.

If the electrolyte composition contains a fluorosulfate (MFSO₃ (M represents an alkali metal and may be the same alkali metal as in M⁺ in the formula (1))), in order to suppress an increase of resistance when the composition is used as a battery, the content of the MFSO₃ is preferably 100 mass ppm to 100000 mass ppm, more preferably 500 mass ppm to 50000 mass ppm and further preferably 1000 mass ppm to 10000 mass ppm.

The electrolyte composition can be used in, e.g., an electrolytic solution of, e.g., a battery or a capacitor, a selective electrophilic fluorinating agent, a photoacid generator, a thermal acid generator, a near-infrared absorbing dye and an antistatic agent. Particularly, the electrolyte composition contains a component excellent in heat resistance and is thus suitable for use in a high temperature environment.

The electrolyte composition may contain unavoidable impurities derived from, e.g., starting materials for individual components and the process. Examples of the impurities include a residual solvent and a halogenated hydrocarbon containing a halogen except fluorine. As the content of the residual solvent, 2000 mass ppm or less is preferable. As the content of the halogenated hydrocarbon, 1000 mass ppm or less is preferable.

The electrolyte composition may be used in a solution-state by dissolving it in a solvent. The solvent is not particularly limited as long as it dissolves the electrolyte composition; for example, a carbonate solvent is mentioned. Examples of the carbonate solvent include linear carbonate esters such as dimethyl carbonate, ethyl methyl carbonate, diethyl carbonate, diphenyl carbonate and methylphenyl carbonate; saturated cyclic carbonates such as ethylene carbonate, propylene carbonate, 2,3-dimethyl ethylene carbonate (carbonic acid 2,3-butanediyl), 1,2-butylene carbonate and erythritan carbonate; and fluorine-containing cyclic carbonates such as fluoroethylene carbonate, 4,5-difluoroethylene carbonate and trifluoropropylene carbonate.

A method for producing a compound of the above formula (1) comprises, for example, a step of heating a solid-state compound of the above formula (2) at 150°C or more. As the compound (2) serving as a raw material, a single compound may be used alone or two or more compounds may be used in combination. Now, the method for producing a compound of the formula (1) will be more specifically described by taking a method for producing a compound of the above formula (3) as an example.

The compound of the above formula (3) can be obtained by heating the corresponding solid-state alkali metal bis(fluorosulfonyl)imide salt. The heating temperature is preferably 150°C or more, more preferably 155°C or more and further preferably 155°C to 160°C. The heating time, which is not particularly limited, is, for example, preferably 30 minutes or more and more preferably one hour to 120 hours.

When a solid-state alkali metal bis(fluorosulfonyl)imide salt was heated to synthesize a compound of the above formula (3), if the salt is heated sufficiently for a long time, almost all amount of the bis(fluorosulfonyl)imide salt can be converted into the compound of the above formula (3); however, the solid-state alkali metal bis(fluorosulfonyl)imide salt can be only partly converted into the compound of the above formula (3). If the salt is partly converted, the solid substance obtained after heating is a mixture containing the alkali metal bis(fluorosulfonyl)imide salt and a compound of the above formula (3). The mixture can be directly used as the above electrolyte composition.

A mechanism for producing the compound of the above formula (3) when a solid-state alkali metal bis(fluorosulfonyl)imide salt was heated, is not clearly known; however, the present inventors consider that the compound of the formula (3) may be produced through the reaction represented by the following reaction formula:

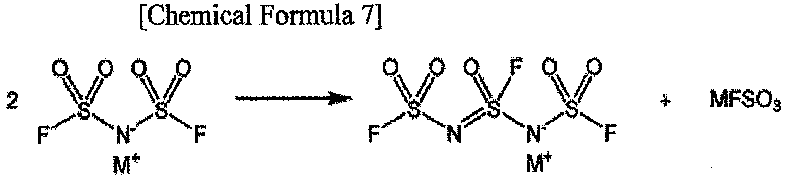

As the bis(fluorosulfonyl)imide salt as a starting material, a commercially available one or a synthesized one may be used. An alkali metal bis(fluorosulfonyl)imide salt can be obtained also by subjecting, for example, ammonium bis(fluorosulfonyl)imide, which is obtained by the reaction between ammonia and a bis(chlorosulfonyl)imide, to a cation exchange reaction using, e.g., a hydroxide salt of an alkali metal.

The solution of the embodiment may contain 0.1 mass ppm to 180000 mass ppm of a compound represented by the following formula (4). Note that, in the specification, the contents of individual components in the solution are expressed by proportions (e.g., mass ppm) to the total amount of the solution, 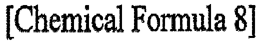 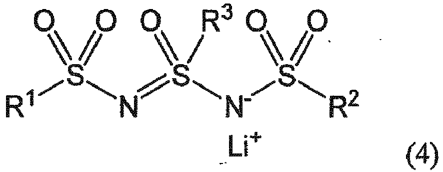 wherein R¹, R² and R³ each individually represent fluorine or a fluoroalkyl group having 1 to 6 carbon atoms.

Examples of the uses of the above solution include an electrolytic solution for, e.g., a battery or a capacitor, a selective electrophilic fluorinating agent, a photoacid generator, a thermal acid generator, a near-infrared absorbing dye and an antistatic agent. Particularly because the above solution contains a component excellent in heat resistance, the solution is suitably applied to applications used in a high temperature environment.

The fluoroalkyl groups having 1 to 6 carbon atoms represented by R¹, R² and R³ may be linear or branched. Note that, the fluoroalkyl group having 1 to 6 carbon atoms may be the one in which the hydrogen atoms of an alkyl group having 1 to 6 carbon atoms are substituted with one or more fluorine atoms and may be a perfluoroalkyl group in which all hydrogen atoms are substituted with fluorine atoms.

The compound in which each R¹, R² and R³ is a fluorine atom is lithium N,N-bis(fluorosulfonyl)imidosulfamoyl fluoride represented by the following formula (5):

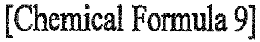

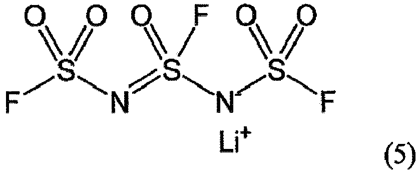

The content of a compound of the above formula (4) in the solution is preferably 1 mass ppm to 20000 mass ppm and more preferably 10 ppm to 10000 mass ppm in order to further increase high-temperature properties of a battery.

The solution can be prepared by dissolving a compound of the formula (4) in a solvent. The solvent is not particularly limited as long as it is used in an electrolytic solution of, e.g., a capacitor and a lithium ion battery. Examples thereof include carbonates such as dimethyl carbonate, ethyl methyl carbonate, ethylene carbonate, diethyl carbonate, propylene carbonate, butylene carbonate and methyl ethyl carbonate; esters such as γ-butyrolactone and methyl formate; and ethers such as 1,2-dimethoxyethane and tetrahydrofuran. These solvents may be used alone or in combination of two types or more.

A compound of the formula (4) may be dissolved alone in a solvent to prepare an electrolytic solution, or may be used in combination with at least one compound selected from the group consisting of LiFSO₃, LiPF₆, LiBF₄ and LiN(SO₂CₙF₂ₙ₊₁)₂ wherein n = an integer of 0 to 6. Of them, LiFSO₃, LiPF₆ or LiN(SO₂F)₂ (lithium (bisfluorosulfonyl)imide, which is also referred to as LiFSI) is preferable. It is preferable that the solution of the embodiment contains 1 mass ppm to 90 mass% of lithium bis(fluorosulfonyl)imide.

If the electrolytic solution contains LiFSO₃, an increase in resistance of a battery can be suppressed, and a decrease in ionic conductivity and an increase in viscosity of the electrolytic solution can be suppressed. In view of these, the content of LiFSO₃ is preferably 0.1 mass ppm to 10000 mass ppm, more preferably 10 mass ppm to 5000 mass ppm and further preferably 100 mass ppm to 2000 mass ppm.

If the electrolytic solution contains LiFSI, ion conductivity is improved and corrosion of a current collector made of, e.g., aluminum can be suppressed. In view of this, the content of LiFSI is preferably 1 mass ppm to 900000 mass ppm, more preferably 10000 mass ppm to 400000 mass ppm and further preferably 30000 mass ppm to 150000 mass ppm.

The compound of the above formula (4) can be obtained by heating a solid-state compound represented by the formula (XSO₂)(XSO₂)NLi. In the compound, X is selected so as to correspond to the above R¹, R² and R³ and a single or two or more compounds represented by (XSO₂)(XSO₂)NLi can be appropriately used depending on the structure of a desired compound represented by the formula (4). For example, the compound of the above formula (5) can be obtained by heating a solid-state LiFSI. As the heating temperature, 150°C or more is preferable, 155°C or more is more preferable and 155°C to 160°C is further preferable. As the heating time, which is not particularly limited, for example, 30 minutes or more is preferable and one hour to 120 hours is more preferable.

In the case where solid-state LiFSI was heated to synthesize the compound of the above formula (5), if the solid substance is heated sufficiently for a long time, almost all amount of the LiFSI can be converted into the compound of the above formula (5); however, a solid-state LiFSI can be only partly converted into the compound of the above formula (5). If the solid substance is partly converted, the solid substance obtained after heating is a mixture (electrolyte composition) containing LiFSI and the compound of the above formula (5). The mixture can be dissolved in a solvent to obtain an electrolytic solution.

A mechanism for producing the compound of the above formula (5) when a solid-state LiFSI was heated, is not clearly known; however, the present inventors consider that the compound of the formula (5) may be produced through the reaction represented by the following reaction formula:

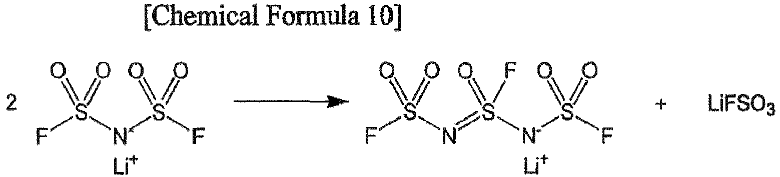

As a lithium bis(fluorosulfonyl)imide salt as a starting material, a commercially available one or a synthesized one may be used. A lithium bis(fluorosulfonyl)imide salt can be obtained also by subjecting, for example, ammonium bis(fluorosulfonyl)imide, which is obtained by the reaction between ammonia and a bis(chlorosulfonyl)imide, to a cation exchange reaction using, e.g., lithium hydroxide.

Since the electrolytic solution of the embodiment is excellent in high-temperature properties such as a cycle capacity retention rate at a high temperature, the electrolytic solution can be suitably used in, e.g., a capacitor and lithium ion battery.

As a specific structure of the lithium ion battery, which is not particularly limited, a general structure can be employed. A positive electrode can contain a positive electrode material commonly used, such as an oxide complex of Li and a transition metal. A negative electrode can contain a negative electrode material commonly used such as graphite, Si or Sn oxide.

### EXAMPLES

Now, the present invention will be more specifically described based on Examples; however, the present invention is not limited to these.

### <Example A>

### [¹⁹F-NMR measurement]

In the following, ¹⁹F-NMR measurement was performed under the following conditions:
Measuring device: VNMRS 600 (manufactured by VARIAN)
Deuterated solvent: acetonitrile-d₃
Analysis concentration: sample concentration; 12 mass%, internal standard; 3.6 mass%
Cumulated number: 16 times

### <Synthesis Example 1A>

### [Fluorosulfonylimide synthesis step (fluorination step)]

In a reaction vessel A (internal volume: 5 L) made of Pyrex (registered trade mark) and equipped with a stirrer, 990 g of butyl acetate was supplied under a nitrogen stream. To the butyl acetate supplied, 110 g (514 mmol) of bis(chlorosulfonyl)imide was added dropwise at room temperature (25°C).

To the butyl acetate solution of bis(chlorosulfonyl)imide obtained, 55.6 g (540 mmol, 1.05 equivalents to bis(chlorosulfonyl)imide) of zinc fluoride was added at a time at room temperature and the solution was stirred at room temperature for 6 hours until zinc fluoride was completely dissolved. Hereinafter, the solution obtained will be referred to as solution A.

### [Cation exchange step 1-Synthesis of ammonium salt]

In reaction vessel B (internal volume: 3 L) made of Pyrex (registered trade mark) and equipped with a stirrer, 297 g (4360 mmol, 8.49 equivalents to bis(chlorosulfonyl)imide) of 25 mass% ammonia water was supplied. While stirring the ammonia water, solution A was added dropwise in reaction vessel B at room temperature. After completion of the dropwise addition of solution A, stirring was terminated. The reaction system was separated into two layers: an aqueous layer and an organic layer. The aqueous layer containing by-products such as zinc chloride was removed to obtain the organic layer, i.e., a butyl acetate solution of ammonium bis(fluorosulfonyl)imide.

Using the organic layer obtained as a sample, ¹⁹F-NMR measurement was carried out (internal standard substance: trifluoromethylbenzene). In the ¹⁹F-NMR chart obtained, a peak (δ56.0 ppm) derived from ammonium bis(fluorosulfonyl)imide was observed. From the ¹⁹F-NMR measurement results, the concentration of ammonium bis(fluorosulfonyl)imide in the organic layer was obtained by the following internal standard method. More specifically, the content of ammonium bis(fluorosulfonyl)imide in the sample was obtained by using the amount of trifluoromethylbenzene added as an internal standard substance and comparing the integral value of the peak derived from trifluoromethylbenzene and the integral value of the peak derived from ammonium bis(fluorosulfonyl)imide. From the content of ammonium bis(fluorosulfonyl)imide obtained and the concentration of the organic layer (sample concentration) in the sample subjected to the ¹⁹F-NMR measurement, the concentration of ammonium bis(fluorosulfonyl)imide in the organic layer was calculated. In this manner, the crude yield of ammonium bis(fluorosulfonyl)imide was calculated as 416 mmol.

### [Cation exchange step 2-Synthesis of lithium salt]

A 15 mass% aqueous lithium hydroxide solution (133 g (834 mmol in terms of Li)) was added such that the amount of lithium to ammonium bis(fluorosulfonyl)imide contained in the organic layer obtained was 2 equivalents and the mixture was stirred at room temperature for 10 minutes. Thereafter, the aqueous layer was removed from the reaction system to obtain the organic layer, i.e., a butyl acetate solution of lithium bis(fluorosulfonyl)imide (hereinafter also referred to as LiFSI).

Using the organic layer obtained as a sample, ICP emission spectroscopy was carried out. As a result, it was confirmed that the protons of fluorosulfonylimide is exchanged with lithium ions.

Using the organic layer obtained as a sample, ¹⁹F-NMR measurement was carried out (internal standard substance: trifluoromethylbenzene). In the ¹⁹F-NMR chart obtained, a peak of LiFSI was observed. The concentration of LiFSI in the organic layer was obtained by the internal standard method mentioned above. The concentration of LiFSI in the organic layer was 7 mass% (yield of organic layer: 994 g, yield of LiFSI: 69.6 g).

### [Concentration step]

Using a rotary evaporator ("REN-1000", manufactured by IWAKI), the solvent was partially distillated under reduced pressure from the organic layer obtained in the above cation exchange step 2 to obtain 162 g of a concentrated LiFSI solution (concentration: 43 mass%).

To a 500 mL-separable flask equipped with a drip funnel, a cooling pipe and a distillate receiver, 162 g of the above concentrated solution was added. Using a vacuum pump, the pressure within the separable flask was reduced to 667 Pa and the separable flask was soaked in an oil bath warmed to 55°C. Subsequently, the concentrated solution in a separable flask was slowly heated while stirring to distill butyl acetate as a reaction solvent from the fluorosulfonylimide synthesis step. The same volume of 1,2,4-trimethylbenzene as the total volume of the solutions collected in the distillate receiver during the time period from initiation of the distillation up to 10 minutes, was added as a poor solvent in the separable flask. Thereafter, the same volume of 1,2,4-trimethylbenzene as the distillation solution was repeatedly added every 10 minutes in the separable flask. In this manner, the reaction solution was further concentrated; at the same time, the mixing ratio of butyl acetate (reaction solvent) and 1,2,4-trimethylbenzene in the system was changed to precipitate a LiFSI crystal. The above operation was repeated until the supernatant in the separable flask became transparent, and thereafter, the flask was cooled to room temperature. The resultant suspension solution of the LiFSI crystal was filtered to obtain the LiFSI crystal. The time from initiation of heating of the concentrated solution to termination of the concentration step was 6 hours and the time taken to initiation of crystal precipitation was 2 hours.

Subsequently, the crystal obtained was washed with a small amount of hexane, transferred to a flat-bottom tray and dried under reduced pressure of 667 Pa at 55°C for 12 hours to obtain a white crystal of LiFSI (yield: 65.4 g).

An acetonitrile solution of the white crystal of LiFSI obtained was prepared. Using the acetonitrile solution as a measurement sample, the compounds contained in the fluorosulfonylimide salt generated was measured by a gas chromatograph mass spectrometer. As a result of the measurement, in the above crystal, 539 mass ppm of butyl acetate and 136 mass ppm of 1,2,4-trimethylbenzene were contained as residual solvents.

Then, 0.1 g of the white crystal of LiFSI obtained was mixed with 9.9 g of ultra-pure water to prepare a 1 mass% aqueous solution thereof. Using the aqueous solution as a measurement sample, the content of chlorine derived from halogenated hydrocarbon contained in the measurement sample was measured by an ICP emission spectroscopic analyzer (ICPE-9000: manufactured by Shimadzu Corporation). Note that, the quantitation limit (lower limit) of the ICP emission spectroscopic analyzer is 0.1 mass ppm.

From the analysis by the gas chromatograph mass spectrometer, it was determined that chlorine detected by the ICP emission spectroscopic analyzer is derived from a halogenated hydrocarbon based on the isotope peaks, molecular weights and fragments. When the (molecular) constitution was confirmed based on the measurement results by the gas chromatograph mass spectrometer, the content of the halogenated hydrocarbon was 35 mass ppm or less and the content of chlorine derived from the halogenated hydrocarbon was 10 mass ppm or less.

### <Synthesis Example 2A>

LiF (14.3 g (0.55 mol)) was weighed and supplied in a reaction vessel made of PFA (fluororesin). While cooling the reaction vessel on ice, 90.47 g (0.50 mol) of liquid-state bis(fluorosulfonyl)imide (HFSI) was added to prepare a slurry-state reaction mixture. When the reaction mixture was heated to 140°C, lithium fluoride was dissolved to obtain a liquid-state reaction solution. The temperature of the reaction solution was maintained at 140°C and a reaction was performed for 15 minutes. The reaction solution was further heated under reduced pressure of 10 hPa at 140°C to 145°C for 2 hours, and thereafter, heated at normal pressure in a nitrogen-stream environment at 140°C for 24 hours. As a result, 72 g of an electrolyte composition was obtained.

### <Example 1A>

One hundred of grams of LiFSI, which was obtained in accordance with the method of Synthesis Example 1A, was supplied in a three-necked flask made of a fluororesin. The flask was heated under nitrogen seal until the internal temperature of the flask reached 150°C, kept for 18 hours and allowed to naturally cool down to obtain 95 g of a solid-state product (hereinafter referred to as product 1A). Using Product 1A as a sample, analysis by ¹⁹F-NMR was carried out (internal standard substance: benzenesulfonyl fluoride, δ68.9 ppm; singlet; hereinafter benzenesulfonyl fluoride was used as the internal standard). As a result, a peak derived from LiFSI (δ55.4 ppm, singlet) and a peak derived from LiFSO₃ (δ40.1 ppm, singlet) and, in addition, peaks respectively at δ64.67 ppm (triplet: J = 7.6Hz) and δ58.77 ppm (doublet: J = 7.6Hz,) were observed. The area ratio of the peak at δ64.67 ppm and the peak at δ58.77 ppm was 1: 2. From these facts, it is considered that the peak at δ64.67 ppm and the peak at δ58.77 ppm are respectively derived from fluorine, which is bonded to S positioned at the center of a compound represented by the above formula (1), i.e., lithium N,N-bis(fluorosulfonyl)imidosulfamoyl fluoride (hereinafter also referred to as LiFSISF) and fluorine (atoms) which are bonded to S at both ends. The content of LiFSISF in product 1A obtained by the above internal standard method was 1.2 mass%. The content of LiFSO₃ obtained in the same manner was 0.8 mass%.

In order to identify the molecular constitution, product 1A was dissolved in ultra-pure water to prepare a 0.5 mass% aqueous solution, which was subjected to analysis by LC-MS. The analysis conditions are as follows.
HPLC: LC 30A System (manufactured by Shimadzu Corporation) Mobile phase: solvent A (0.1 mass% aqueous formic acid solution),
solvent B (methanol), A:B=20:80 (volume ratio)
Flow velocity: 0.1 ml/min
Column temperature: 40°C
Detector: absorbance detector (190 nm to 800 nm)
Injection volume: 1 µL
MS: Q Exactive (manufactured by Thermo Fisher Scientific)
Ionization method: electrospray ionization method (ESI)
Probe heater temperature: 330°C
Capillary temperature: 330°C

In LC-MS, peak separation was not performed and all components were ionized. Of the components ionized in the first stage, major peaks were subjected to MS/MS measurement. From the patterns of fragment ions, anions of LiFSISF were identified.

As a result of mass analysis by the ESI method, a molecular ion peak was detected at m/z = 260.89. As s a result of MS/MS measurement, fragment ions were detected at m/z = 96.96 (O₂NFS) and m/z = 177.93 (O₃N₂F₂S₂). The structure formula satisfying generation of these fragment ions is an anion represented by the formula (3).

To check the concentration of Li element in product 1A, 0.01 g of product 1A was diluted with 4 mass% nitric acid up to 50000 fold to prepare a measurement solution. The concentration of Li element in product 1A was checked by an ICP emission spectroscopic analyzer (ICPE-9000: manufactured by Shimadzu Corporation). As a result, Li alone was detected as a metal component. The concentration of Li element per product 1A (1 g) was 5.5 mmol. The molar number of Li detected was almost equivalent to the total of molar numbers of LiFSI, LiFSO₃ and LiFSISF calculated from analysis results by ¹⁹F-NMR.

From the above ICP emission spectroscopic analysis results, it was found that the metal ion contained in product 1A is Li.

### <Example 2A>

Cation exchange step 2 was carried out in the same manner as in Synthesis Example 1A except that 222 g (833 mmol in terms of Na) of a 15 mass% aqueous sodium hydroxide solution was used in place of lithium hydroxide in cation exchange step 2 of Synthesis Example 1A to prepare a sodium bis(fluorosulfonyl)imide (NaFSI) solution.

Then, concentration step and purification were carried out in the same manner as in Synthesis Example 1A except that decane was used as a poor solvent; the degree of vacuum was changed to 1.33 kPa (10 torr); and the heating temperature was changed to 60°C to obtain a white crystal of NaFSI.

One hundred grams of NaFSI obtained in accordance with the above method was supplied in a three-necked flask made of a fluororesin. The flask under nitrogen seal was heated until the internal temperature of the flask reached 150°C, kept for 18 hours and allowed to naturally cool down to obtain 95 g of a solid-state product (hereinafter referred to as product 2A). Product 2A was subjected to ¹⁹F-NMR measurement. As a result, it was confirmed that NaFSISF was generated. The content of NaFSISF obtained by the internal standard method was 1.3 mass%. The content of NaFSO₃ obtained in the same manner was 0.8 mass%.

### <Example 3A>

Cation exchange step 2 was carried out in the same manner as in Synthesis Example 1A except that 312 g (834 mmol in terms of K) of a 15 mass% aqueous potassium hydroxide solution was used in place of lithium hydroxide in Cation exchange step 2 of Synthesis Example 1A cation exchange step 2 to obtain a potassium bis(fluorosulfonyl)imide (KFSI) solution.

Then, concentrated step and purification were carried out in the same manner as in Synthesis Example 1A except that decane was used as a poor solvent; the degree of vacuum was changed to 4 kPa (30 torr); and the heating temperature was changed to 60°C to obtain a white crystal of KFSI.

One hundred grams of KFSI obtained in accordance with the above method was supplied in a three-necked flask made of a fluororesin. The flask under nitrogen seal was heated until the internal temperature of the flask reached 150°C, kept for 18 hours and allowed to naturally cool down to obtain 96 g of a solid-state product (hereinafter referred to as product 3A). Product 3A was subjected to ¹⁹F-NMR measurement to confirm that KFSISF was generated. The content of KFSISF obtained by the internal standard method was 1.4 mass%. The content of KFSO₃ obtained in the same manner was 0.9 mass%.

### <Example 4A>

One hundred grams of LiFSI obtained in accordance with the method of Synthesis Example 1A was supplied in a three-necked flask made of a fluororesin. The flask under nitrogen seal was heated until the internal temperature of the flask reached 150°C, kept for 2 hours and allowed to naturally cool down to obtain 95.4 g of a solid-state product (hereinafter referred to as product 4A).

### <Example 5A>

One hundred grams of LiFSI obtained in accordance with the method of Synthesis Example 1A was supplied in a three-necked flask made of a fluororesin. The flask under nitrogen seal was heated until the internal temperature of the flask reached 150°C, kept for 108 hours and allowed to naturally cool down to obtain 95.2 g of a solid-state product (hereinafter referred to as product 5A).

### <Comparative Example 1A>

One hundred grams of LiFSI obtained in accordance with the method of Synthesis Example 1A was supplied in a three-necked flask made of a fluororesin. The flask under nitrogen seal was heated until the internal temperature of the flask reached 145°C, kept for 10 minutes and allowed to naturally cool down to obtain 95.2 g of a solid-state product (hereinafter referred to as product C1A).

### <Comparative Example 2A>

After LiFSI was obtained in accordance with the method of Synthesis Example 1A, no treatment was applied (hereinafter referred to as product C2A).

Products 4A, 5A, C1A and C2A were individually analyzed by ¹⁹F-NMR to obtain concentrations of individual components in accordance with the internal standard method. The results are shown in Table 1. Note that, in Table 1, N.D. indicates that a target component was not detected.

**[Table 1]**

| | Example 4A | Example 5A | Comparative Example 1A | Comparative Example 2A |
|---|---|---|---|---|
| Heating conditions | 150°C 2 h | 150°C 108 h | 145°C 10 min | None |
| LiFSI/mass% | 95.9 | 90.4 | 97.4 | 99.3 |
| LiFSISF/mass ppm | 1400 | 67500 | N.D. | N.D. |
| LiFSO₃/mass ppm | 6900 | 35700 | 3800 | N.D. |

LiPF₆ and each of products 4A, 5A, C1A and C2A were individually dissolved in a solvent (EC:MEC = 3:7 (mass ratio)) to prepare electrolytic solutions 4A, 5A, C1A and C2A having a volume of 10 mL. The concentration of LiPF₆ in electrolytic solutions 4A, 5A, C1A and C2A was 0.6 M and products 4A, 5A, C1A and C2A were added to the respective electrolytic solutions such that the concentrations of FSI anion were 0.6 M. As LiPF₆, a commercially available product was used. The actual weighed values of the electrolyte compositions and the contents of LiFSISF and LiFSO₃ in the electrolytic solution calculated from the analysis results of Table 1 are shown in Table 2.

**[Table 2]**

| | Electrolytic solution 4A | Electrolytic solution 5A | Electrolytic solution C1A | Electrolytic solution C2A |
|---|---|---|---|---|
| Product (weighed value/g) | 4A (1.17) | 5A (1.221) | C1A (1.172) | C2A (1.168) |
| LiFSISF/mass ppm | 135 | 6774 | N.D. | N.D. |
| LiFSO₃/mass ppm | 664 | 3583 | 366 | N.D. |

Using electrolytic solution 5A as a sample, ¹⁹F-NMR measurement was carried out. Using the measurement results, the content of LiFSISF was obtained in accordance with the internal standard method as mentioned above. The content of LiFSISF was 6800 mass ppm, which was substantially the same value as the calculated value shown in Table 2.

Using electrolytic solutions 4A, 5A, C1A and C2A, battery cells 4A, 5A, C1A and C2A were fabricated. Evaluation of the high-temperature performance of the battery cells was carried out. As the battery cells used in high-temperature performance evaluation, a laminated battery cell of 1000 mAh design using LiNi_{0.5}Co_{0.2}Mn_{0.3}O₂ as the positive electrode, graphite as the negative electrode and a polyethylene (PE) separator, was used.

The battery cells according to the aforementioned specifications were aged in the following conditions to accomplish battery cells 4A, 5A, C1A and C2A.

### [Aging condition]

Into the above battery cells, electrolytic solutions 4A, 5A, C1A and C2A were injected. One hour later, the cells were charged with a constant current/constant voltage of 4.2 V/200 mA for 90 minutes and then allowed to stand still for 72 hours. Thereafter, extra laminate was opened and the cells were subjected again to welding under vacuum. In this manner, gas was released. After the release of gas, charge with a constant current/constant voltage of 4.2 V/500 mA was carried out for 5 hours, an interval of 10 minutes was provided, and then, discharge (a constant current of 200 mA) was carried out up to a closed circuit voltage of 2.75 V. After an interval of 10 minutes, charge with a constant current/constant voltage charge of 4.2 V/500 mA was further carried out for 5 hours. After an interval of 10 minutes, discharge (a constant current of 1000 mA) was carried out up to a closed circuit voltage of 2.75 V. The aging was entirely carried out at 25°C.

### [Cycle characteristics at 45°C]

Battery cells 4A, 5A, C1A and C2A were each subjected to a charge and discharge cycle test performed in the following conditions. The cycle was repeated 500 times.

Charge conditions: charge with a constant voltage/current of 4.2 V/1000 mA at 45°C was carried out until the current value reached 20 mA, and thereafter, the operation was ceased at 45°C for 10 minutes.

Discharge conditions: discharge (a constant current of 1000 mA) at 45°C was carried out until a closed circuit voltage reached 2.75 V, and thereafter, the operation was ceased at 45°C for 10 minutes.

After completion of 500 cycles, charge with constant current/constant voltage of 4.2 V/1000 mA was carried out in a 25°C environment until the current value reached 20 mA. The operation was ceased at 25°C for 10 minutes. Thereafter, discharge (20 mA), at which resistance can be ignored, was carried out in a 25°C environment until the closed circuit voltage reached 2.75 V. After completion of discharge, the ratio of the discharge capacity obtained to the initial discharge capacity (the first discharge capacity after aging) was calculated as a capacity retention rate. The results are shown in Table 3.

**[Table 3]**

| | Battery cell 4A | Battery cell 5A | Battery cell C1A | Battery cell C2A |
|---|---|---|---|---|
| Capacity retention rate/% | 76.7 | 77.5 | 75.6 | 75.5 |
| 20 mA discharge capacity/mAh | 787.8 | 796.7 | 773 | 774.4 |

Battery cells 4A and 5A containing LiFSISF had a higher capacity retention rate at a high temperature cycle than the battery cells C1A and C2A not containing LiFSISF. In addition, the battery cells 4A and 5A had a higher "20 mA discharge capacity" after the cycles than the battery cells C1A and C2A. From these, it is found that the amount of Li deposited on a negative electrode to be inactivated is low.

Battery cell C1A containing LiFSO₃ and battery cell C2A not containing LiFSO₃ were almost equivalent in capacity retention rate and "20 mA discharge capacity". Battery cell 5A large in content of LiFSISF was more excellent in capacity retention rate and "20 mA discharge capacity" than battery cell 4A. As described above, battery cells 4A and 5A were improved in battery characteristics after the high temperature cycle. This is conceivably because LiFSISF is excellent in heat resistance and suppresses generation of reaction by-products of the electrolytic solution.

### <Example B>

### [¹⁹F-NMR measurement]

¹⁹F-NMR measurement in the following was carried out in the following conditions.
Measuring device: VNMRS 600 (manufactured by VARIAN)
Deuterated solvent: acetonitrile-d₃
Analysis concentration: sample concentration; 12 mass%, internal standard; 3.6 mass%
Cumulated number: 16

### <Synthesis Example 1B>

### [Fluorosulfonylimide synthesis step (fluorination step)]

In reaction vessel A (internal volume: 5 L) made of Pyrex (registered trade mark) and equipped with a stirrer, 990 g of butyl acetate was supplied under a nitrogen stream. To the butyl acetate supplied, 110 g (514 mmol) of bis(chlorosulfonyl)imide was added dropwise at room temperature (25°C).

To the butyl acetate solution of bis(chlorosulfonyl)imide obtained, 55.6 g (540 mmol, 1.05 equivalents to bis(chlorosulfonyl)imide) of zinc fluoride was added. The mixture was stirred at room temperature for 6 hours until the zinc fluoride was completely dissolved. The solution obtained is called as Solution A.

### [Cation exchange step 1-Synthesis of ammonium salt]

In reaction vessel B (internal volume: 3 L) made of Pyrex (registered trade mark) and equipped with a stirrer, 297 g (4360 mmol, 8.49 equivalents to bis(chlorosulfonyl)imide) of 25 mass% ammonia water was supplied. While stirring the ammonia water, solution A wad added dropwise in reaction vessel B at room temperature. After completion of dropwise addition of solution A, stirring was terminated. The reaction system was separated into two layers: an aqueous layer and an organic layer. The aqueous layer containing by-products such as zinc chloride was removed and the organic layer, i.e., a butyl acetate solution of ammonium bis(fluorosulfonyl)imide was obtained.

Using the organic layer obtained as a sample, ¹⁹F-NMR measurement was carried out (internal standard substance: trifluoromethylbenzene). In the ¹⁹F-NMR chart obtained, a peak (δ56.0 ppm) derived from ammonium bis(fluorosulfonyl)imide was observed. From the ¹⁹F-NMR measurement results, the concentration of ammonium bis(fluorosulfonyl)imide in the organic layer was obtained by the following internal standard method. More specifically, the content of ammonium bis(fluorosulfonyl)imide in the sample was obtained by using the amount of trifluoromethylbenzene added as an internal standard substance and comparing the integral value of the peak derived from trifluoromethylbenzene and the integral value of the peak derived from ammonium bis(fluorosulfonyl)imide. From the content of ammonium bis(fluorosulfonyl)imide obtained and the concentration of the organic layer (sample concentration) in the sample subjected to the ¹⁹F-NMR measurement, the concentration of ammonium bis(fluorosulfonyl)imide in the organic layer was calculated. In this manner, the crude yield of ammonium bis(fluorosulfonyl)imide was calculated as 416 mmol.

### [Cation exchange step 2-synthesis of lithium salt]

A 15 mass% aqueous lithium hydroxide solution (133 g (834 mmol in terms of Li)) was added such that the amount of lithium to ammonium bis(fluorosulfonyl)imide contained in the organic layer obtained was 2 equivalents and the mixture was stirred at room temperature for 10 minutes. Thereafter, the aqueous layer was removed from the reaction system to obtain the organic layer, i.e., a butyl acetate solution of lithium bis(fluorosulfonyl)imide. Note that, hereinafter, lithium bis(fluorosulfonyl)imide will be also referred to as LiFSI.

Using the organic layer obtained as a sample, ICP emission spectroscopy was carried out. As a result, it was confirmed that the protons of fluorosulfonylimide are exchanged with lithium ions.

Using the organic layer obtained as a sample, ¹⁹F-NMR measurement was carried out (internal standard substance: trifluoromethylbenzene). In the ¹⁹F-NMR chart obtained, a peak of LiFSI was observed. The concentration of LiFSI in the organic layer was obtained by the internal standard method mentioned above. The concentration of LiFSI in the organic layer was 7 mass% (yield of organic layer: 994 g, yield of LiFSI: 69.6 g).

### [Concentrated step]

Using a rotary evaporator ("REN-1000", manufactured by IWAKI), the solvent was partially distillated under reduced pressure from the organic layer obtained in the above cation exchange step 2 to obtain 162 g of a concentrated LiFSI solution (concentration: 43 mass%).

In a 500 mL-separable flask equipped with a drip funnel, a cooling pipe and a distillate receiver, 162 g of the above concentrated solution was added. Using a vacuum pump, the pressure within the separable flask was reduced up to 667 Pa and the separable flask was soaked in an oil bath warmed to 55°C. Subsequently, the concentrated solution in a separable flask was slowly heated while stirring to distill butyl acetate as a reaction solvent from the fluorosulfonylimide synthesis step. The same volume of 1,2,4-trimethylbenzene as the total volume of the solutions collected in the distillate receiver during the time period from initiation of the distillation up to 10 minutes, was added as a poor solvent in the separable flask. Thereafter, the same volume of 1,2,4-trimethylbenzene as the distillation solution was repeatedly added every 10 minutes in the separable flask. In this manner, the reaction solution was further concentrated; at the same time, the mixing ratio of butyl acetate (reaction solvent) and 1,2,4-trimethylbenzene in the system was changed to precipitate a LiFSI crystal. The above operation was repeated until the supernatant in the separable flask became transparent and the flask was cooled up to room temperature. The resultant suspension solution of the LiFSI crystal was filtered to obtain the LiFSI crystal. Note that, the time from initiation of heating of the above concentrated solution to termination of the concentration step was 6 hours and the time required until initiation of crystal precipitation was 2 hours.

Then, the crystal obtained was washed with a small amount of hexane, transferred to a flat-bottom tray and dried under reduced pressure of 667 Pa at 55°C for 12 hours to obtain a white crystal of LiFSI (yield: 65.4 g).

An acetonitrile solution of the white crystal of LiFSI obtained was prepared. Using the acetonitrile solution as a measurement sample, the compounds contained in the fluorosulfonylimide salt generated was measured by a gas chromatograph mass spectrometer. As a result of the measurement, in the above crystal, 539 mass ppm of butyl acetate and 136 mass ppm of 1,2,4-trimethylbenzene were contained as residual solvents.

Then, 0.1 g of the white crystal of LiFSI obtained was mixed with 9.9 g of ultra-pure water to prepare an aqueous solution thereof with 1 mass% concentration. Using the aqueous solution as a measurement sample, the content of chlorine derived from halogenated hydrocarbon contained in the measurement sample was measured by an ICP emission spectroscopic analyzer (ICPE-9000: manufactured by Shimadzu Corporation). Note that, the quantitation limit (lower limit) of the ICP emission spectroscopic analyzer is 0.1 mass ppm.

From the analysis by the gas chromatograph mass spectrometer, it was determined that chlorine detected by the ICP emission spectroscopic analyzer is derived from a halogenated hydrocarbon based on the isotope peaks, molecular weights and fragments. When the (molecular) constitution was confirmed based on the measurement results by the gas chromatograph mass spectrometer, the content of the halogenated hydrocarbon was 35 mass ppm or less and the content of chloride derived from the halogenated hydrocarbon was 10 mass ppm or less.

### <Synthesis Example 2B>

LiF (14.3 g (0.55 mol)) was weighed and supplied in a reaction vessel made of PFA (fluororesin). While cooling the reaction vessel on ice, 90.47 g (0.50 mol) of liquid-state bis(fluorosulfonyl)imide (HFSI) was added to prepare a slurry-state reaction mixture. When the reaction mixture was heated to 140°C, lithium fluoride was dissolved to obtain a liquid-state reaction solution. The temperature of the reaction solution was maintained at 140°C and a reaction was performed for 15 minutes. The reaction solution was heated under reduced pressure of 10 hPa at 140°C to 145°C for 2 hours, and thereafter, heated at normal pressure in a nitrogen-stream environment at 140°C for 24 hours. As a result, 72 g of an electrolyte composition was obtained.

### <Example 1B>

One hundred grams of LiFSI obtained in accordance with the method of Synthesis Example 1B was supplied in a reaction vessel made of PFA (fluororesin). A thermometer was inserted in the interior, the vessel was heated until the temperature of a melt within the vessel reached 150°C under a nitrogen stream and heated for 2 hours so as to keep 150°C. Thereafter, the vessel was allowed to naturally cool down to obtain 95.4 g of an electrolyte composition. Using the electrolyte composition as a sample, analysis by ¹⁹F-NMR was performed (internal standard substance: benzenesulfonyl fluoride, δ68.9 ppm, singlet, hereinafter benzenesulfonyl fluoride was used as the internal standard). As a result, a peak derived from LiFSI (δ55.4 ppm, singlet) and a peak derived from LiFSO₃ (δ40.1 ppm, singlet) and, in addition, peaks respectively at δ64.67 ppm (triplet: J = 7.6Hz) and δ58.77 ppm (doublet: J = 7.6Hz,) were observed. The area ratio of the peak at δ64.67 ppm and the peak at δ58.77 ppm was 1:2. From these facts, it is considered that the peak at δ64.67 ppm and the peak at δ58.77 ppm are respectively derived from fluorine, which is bonded to S positioned at the center of a compound represented by the above formula (4), i.e., lithium N,N-bis(fluorosulfonyl)imidosulfamoyl fluoride (hereinafter also referred to as LiFSISF) and fluorine (atoms), which are bonded to S at both ends. The content of LiFSISF in the electrolyte composition obtained by the internal standard method was 1400 mass ppm. The content of LiFSO₃ obtained in the same manner as above was 6900 mass ppm.

### <Example 2B>

One hundred grams of LiFSI obtained by the method of Synthesis Example 1B was supplied in a reaction vessel made of PFA (fluororesin). A thermometer was inserted in the interior, the vessel was heated until the temperature of a melt within the vessel reached 150°C under a nitrogen stream and further heated for 108 hours so as to keep 150°C. Thereafter, the vessel was allowed to naturally cool down to obtain 95.2 g of an electrolyte composition.

### <Comparative Example 1B>

One hundred grams of LiFSI obtained by the method of Synthesis Example 1B was supplied in a reaction vessel made of PFA (fluororesin). A thermometer was inserted in the interior, the vessel was heated until the temperature of a melt within the vessel reached 145°C under a nitrogen stream and further heated for 10 minutes so as to keep 145°C. Thereafter, the vessel was allowed to naturally cool down to obtain 95.6 g of an electrolyte composition.

Electrolyte compositions of Examples 1B, 2B and Comparative Example 1B were individually analyzed by ¹⁹F-NMR and the content of individual components were obtained by the internal standard method mentioned above. The results are shown in Table 4. In Comparative Example 2B shown in Table 4, LiFSI obtained by the method of Synthesis Example 1B was directly subjected to ¹⁹F-NMR analysis without applying a heat treatment.

**[Table 4]**

| | Example 1B | Example 2B | Comparative Example 1B | Comparative Example 2B |
|---|---|---|---|---|
| Heating conditions | 150°C 2 h | 150°C 108 h | 145°C 10 min | None |
| LiFSI/mass% | 95.9 | 90.4 | 97.4 | 99.3 |
| LiFSISF/mass ppm | 1400 | 67500 | N.D. | N.D. |
| LiFSO₃/mass ppm | 6900 | 35700 | 3800 | N.D. |

LiPF₆ and the electrolyte compositions of Examples 1B, 2B, Comparative Examples 1B and 2B were individually dissolved in a solvent (EC:MEC = 3:7 (volume ratio)) to prepare electrolytic solutions 1B, 2B, C1B and C2B having a volume of 10 mL. The concentration of LiPF₆ in electrolytic solutions 1B, 2B, C1B and C2B was 0.6 M and the electrolyte compositions of Examples 1B, 2B, Comparative Examples 1B and 2B were added to the respective electrolytic solutions such that the concentration of FSI anion became 0.6 M. As LiPF₆, a commercially available product was used.

The actual weighed values of the electrolyte compositions and the contents of LiFSISF and LiFSO₃ in the electrolytic solutions calculated from the analysis results of Table 4 are shown in Table 5.

**[Table 5]**

| | Electrolytic solution 1B | Electrolytic solution 2B | Electrolytic solution C1B | Electrolytic solution C2B |
|---|---|---|---|---|
| Electrolyte composition/g | Example 1B 1.17 | Example 2B 1.221 | Comparative Example 1B 1.172 | Comparative Example 2B 1.168 |
| LiFSISF/mass ppm | 135 | 6774 | N.D. | N.D. |
| LiFSO₃/mass ppm | 664 | 3583 | 366 | N.D. |

Using electrolytic solution 2B as a sample, ¹⁹F-NMR measurement was carried out. Using the measurement results, the content of LiFSISF was obtained in accordance with the internal standard method as mentioned above. The content of LiFSISF was 6800 mass ppm, which was substantially the same value as the calculated value shown in Table 5.

Using electrolytic solutions 1B, 2B, C1B and C2B, battery cells 1B, 2B, C1B and C2B were fabricated. The high-temperature performance evaluation of the battery cells was carried out As the battery cells used in high-temperature performance evaluation, a laminated battery cell of 1000 mAh design using LiNi_{0.5}Co_{0.2}Mn_{0.3}O₂ as the positive electrode, graphite as the negative electrode and a polyethylene (PE) separator, was used.

The battery cells according to the aforementioned specifications were aged in the following conditions to accomplish battery cells 1B, 2B, C1B and C2B.

### [Aging condition 1]

Into the above battery cells, electrolytic solutions 1B, 2B, C1B and C2B were injected. One hour later, the cells were charged with a constant current/constant voltage of 4.2 V/200 mA for 90 minutes and then allowed to stand still for 72 hours. Thereafter, extra laminate was opened and the cells were subjected again to welding under vacuum. In this manner, gas was released. After the release of gas, charge with constant current/constant voltage with 4.2 V/500 mA was carried out for 5 hours, an interval of 10 minutes was provided, and then, discharge (a constant current of 200 mA) was carried out up to a closed circuit voltage of 2.75 V. After an interval of 10 minutes, charge with a constant current/constant voltage of 4.2 V/500 mA was further carried out for 5 hours. With an interval of 10 minutes, discharge (a constant current of 1000 mA) was carried out up to a closed circuit voltage of 2.75 V. The aging was entirely carried out in a 25°C environment

### [Cycle characteristics 1 at 45°C]

Battery cells 1B, 2B, C1B and C2B were each subjected to a charge and discharge cycle test performed in the following conditions. The cycle was repeated 500 times.
Charge conditions: charge with a constant voltage/current of 4.2 V/1000 mA at 45°C was carried out until the current value reached 20 mA, and thereafter, the operation was ceased at 45°C for 10 minutes.
Discharge conditions: discharge (a constant current of 1000 mA) at 45°C was carried out until a closed circuit voltage reached 2.75 V, and thereafter, the operation was ceased at 45°C for 10 minutes.

After completion of 500 cycles, charge with a constant current/constant voltage of 4.2 V/1000 mA was carried out in a 25°C environment until the current value reached 20 mA. The operation was ceased at 25°Cfor 10 minutes. Thereafter, discharge (20 mA), at which resistance can be ignored, was carried out at 25°Cuntil the closed circuit voltage reached 2.75 V. After completion of discharge, the ratio of the discharge capacity obtained to the initial discharge capacity (the first discharge capacity after aging) was calculated as a capacity retention rate. The results are shown in Table 6.

**[Table 6]**

| | Battery cell 1B | Battery cell 2B | Battery cell C1B | Battery cell C2B |
|---|---|---|---|---|
| Capacity retention rate/% | 76.7 | 77.5 | 75.6 | 75.5 |
| 20 mA discharge capacity/mAh | 787.8 | 796.7 | 773.0 | 774.4 |

Battery cells 1B and 2B containing LiFSISF had a higher capacity retention rate at a high temperature cycle than the battery cells C1B and C2B not containing LiFSISF. In addition, the battery cells 1B and 2B had a higher "20 mA discharge capacity" after the cycles than the battery cells C1B and C2B. From these, it is found that the amount of Li deposited on a negative electrode and inactivated is low.

Battery cell C1B containing LiFSO₃ and battery cell C2B not containing LiFSO₃ were equivalent in capacity retention rate and "20 mA discharge capacity". Battery cell 2B large in content of LiFSISF was more excellent in capacity retention rate and "20 mA discharge capacity" than battery cell 1B. From these, it is considered that the effect of improving cycle capacity retention rate at a high temperature was obtained by adding LiFSISF in the electrolytic solution.

### <Example 3B>

One hundred grams of LiFSI obtained in accordance with the method of Synthesis Example 1B was supplied in a reaction vessel made of PFA (fluororesin). A thermometer was inserted in the interior, the vessel was heated until the temperature of a melt within the vessel reached 150°C under a nitrogen stream and heated for 10 minutes so as to keep 150°C. Thereafter, the vessel was allowed to naturally cool down to obtain 95.6 g of an electrolyte composition.

### <Example 4B>

One hundred grams of LiFSI obtained in accordance with the method of Synthesis Example 1B was supplied in a reaction vessel made of PFA (fluororesin). A thermometer was inserted in the interior, the vessel was heated until the temperature of a melt within the vessel reached 150°C under a nitrogen stream and heated for 30 minutes so as to keep 150°C. Thereafter, the vessel was allowed to naturally cool down to obtain 95.5 g of an electrolyte composition.

### <Example 5B>

One hundred grams of LiFSI obtained in accordance with the method of Synthesis Example 1B was supplied in a reaction vessel made of PFA (fluororesin). A thermometer was inserted in the interior, the vessel was heated until the temperature of a melt within the vessel reached 150°C under a nitrogen stream and heated for 17 hours so as to keep 150°C. Thereafter, the vessel was allowed to naturally cool down to obtain 95.4 g of an electrolyte composition.

### <Example 6B>

One hundred grams of LiFSI obtained in accordance with the method of Synthesis Example 1B was supplied in a reaction vessel made of PFA (fluororesin). A thermometer was inserted in the interior, the vessel was heated until the temperature of a melt within the vessel reached 150°C under a nitrogen stream and heated for 36.5 hours so as to keep 150°C. Thereafter, the vessel was allowed to naturally cool down to obtain 95.0 g of an electrolyte composition.

Electrolyte compositions of Examples 3B to 6B were individually analyzed by ¹⁹F-NMR and the content of individual components were obtained by the internal standard method mentioned above. The results are shown in Table 7.

**[Table 7]**

| | Example 3B | Example 4B | Example 5B | Example 6B | Comparative Example 2B |
|---|---|---|---|---|---|
| Heating time | 150°C 10 min | 150°C 30 min | 150°C 17 h | 150°C 36.5 h | None |
| LiFSI/mass % | 99.31 | 99.55 | 98.74 | 96.83 | 99.3 |
| LiFSISF/mass ppm | 100 | 300 | 10900 | 23100 | N.D. |
| LiFSO₃ /mass ppm | 2500 | 2500 | 1800 | 14100 | N.D. |

LiPF₆ and the electrolyte compositions of Examples 3B to 6B were individually dissolved in a solvent (EC:MEC = 3:7 (volume ratio)) to prepare electrolytic solutions 3B to 6B having a volume of 10 mL. The concentration of LiPF₆ in electrolytic solutions 3B to 6B was 0.6 M and the electrolyte compositions of Examples 3B to 6B were added to respective electrolytic solutions such that the concentration of FSI anion became 0.6 M. Note that, as LiPF₆, a commercially available product was used.

The actual weighed values of the electrolyte compositions and the contents of LiFSISF and LiFSO₃ in the electrolytic solutions calculated from the analysis results of Table 7 are shown in Table 8.

**[Table 8]**

| | Electrolytic solution 3B | Electrolytic solution 4B | Electrolytic solution 5B | Electrolytic solution 6B | Electrolytic solution C2B |
|---|---|---|---|---|---|
| Electrolyte composition/g | 1.132 | 1.127 | 1.119 | 1.142 | 1.168 |
| LiFSISF/mass ppm | 9 | 28 | 1003 | 2168 | N.D. |
| LiFSO₃/mass ppm | 233 | 232 | 166 | 1324 | N.D. |

Using electrolytic solution 6B as a sample, ¹⁹F-NMR measurement was carried out. Using the measurement results, the content of LiFSISF was obtained in accordance with the internal standard method as mentioned above. The content of LiFSISF was 2200 mass ppm, which was substantially the same value as the calculated value shown in Table 8.

Using electrolytic solutions 3B to 6B and electrolytic solution C2B, battery cells 3B to 6B and C3B were prepared. The high-temperature performance evaluation of the battery cells was carried out. As the battery cells used in high-temperature performance evaluation, a laminated battery cell of 30 mAh design using LiNi_{1/3}Co_{1/3}N4n_{1/3}O₂ as the positive electrode, graphite as the negative electrode and a polyethylene (PE) separator, was used.

The battery cells according to the aforementioned specifications were aged in the following conditions to accomplish battery cells 3B to 6B and C3B.

### [Aging condition 2]

Into the above battery cells, electrolytic solutions 3B to 6B and C2B were injected. One hour later, the cells were charged with a constant current/constant voltage of 4.2 V/6 mA for 90 minutes and then allowed to stand still for 72 hours. Thereafter, extra laminate was opened and the cells were subjected again to welding under vacuum. In this manner, gas was released. After the release of gas, charge with a constant current/constant voltage of 4.2 V/15 mA was carried out for 5 hours, an interval of 10 minutes was provided, and then, discharge (a constant current of 6 mA) was carried out up to a closed circuit voltage of 2.75 V. After an interval of 10 minutes, constant current/constant voltage charge with 4.2 V/15 mA was further carried out for 5 hours. With an interval of 10 minutes, discharge (a constant current of 30 mA) was carried out up to a closed circuit voltage of 2.75 V. The aging was entirely carried out at 25°C.

### [Cycle characteristics 2 at 45°C]

Battery cells 3B to 6B and C3B were each subjected to a charge and discharge cycle test performed in the following conditions. The cycle was repeated to 1000 times.
Charge conditions: constant voltage/current charge with 4.2 V/30 mA was carried out at 45°C until the current value reached 0.6 mA, and thereafter, the operation was ceased at 45°C for 10 minutes.
Discharge conditions: constant current discharge (30 mA) at 45°C until a closed circuit voltage reached 2.75 V, and thereafter, the operation was ceased at 45°C for 10 minutes.

The ratio of discharge capacity at 1000th cycle relative to that of the first cycle was calculated and regarded as the capacity retention rate. The results are shown in Table 9.

**[Table 9]**

| | Battery cell 3B | Battery cell 4B | Battery cell 5B | Battery cell 6B | Battery cell C3B |
|---|---|---|---|---|---|
| Capacity retention rate/% | 76.7 | 77.5 | 77.8 | 78.0 | 76.5 |

The content of LiFSISF (contained) in the electrolytic solution increased in the order of battery cells 3B to 6B and the capacity retention rate after the 1000th cycle increased in this order. When battery cells 3B and 4B are compared, the LiFSO₃ contents of them are equivalent; however, battery cell 4B containing a larger amount of LiFSISF shows a satisfactory result with respect to the capacity retention rate. From these, it is considered that an effect of improving a cycle capacity retention rate at a high temperature was obtained by adding LiFSISF.

### [Measurement of initial discharge capacity]

After completion of aging, the capacity of the battery cell was checked at 25°C in the following conditions.
Discharge: constant current discharge (6 mA) was carried out until the closed circuit voltage reached 2.75V, and thereafter, the operation was ceased for 10 minutes.
Charge: constant current/constant voltage charge with 4.2V and 30 mA was carried out until the current value reached 0.6 mA, and thereafter, the operation was ceased for 10 minutes.
Discharge: constant current discharge (30 mA) was carried out until the closed circuit voltage reached 2.75V, and a discharge capacity was measured.

The discharge capacity thus obtained was regarded as the initial discharge capacity.

### [Trickle charge cycle at 45°C]

After the initial discharge capacities of battery cells 3B to 6B and C3B were measured, a trickle charge cycle was repeated 300 times in the following conditions.

### <Cycle conditions from 1 to 100th cycle>

Charge: constant current/constant voltage charge with 4.2 V/30 mA was carried out at 45°C until the current value reached 6 mA, and thereafter, the operation was ceased for 3 hours.

Discharge: constant current discharge (15 mA) was carried out at 45°C for 10 minutes, and thereafter, the operation was ceased for 5 minutes.

### <Cycle conditions from 101st to 300th cycle>

Charge: constant current/constant voltage charge with 4.35 V/30 mA was carried out at 45°C until the current value reached 6 mA, and thereafter, the operation was ceased for 3 hours.

Discharge: constant current discharge (15 mA) was carried out at 45°C for 10 minutes, and thereafter, the operation was ceased for 5 minutes.

After 300 trickle charge cycles were carried out at 45°C in the above conditions, the discharge capacity (after the 300 cycles) was measured in the same manner as in the initial capacity measurement.

The capacity retention rates after 300 cycles are shown in Table 10.

**[Table 10]**

| | Battery cell 3B | Battery cell 4B | Battery cell 5B | Battery cell 6B | Battery cell C3B |
|---|---|---|---|---|---|
| Capacity retention rate/% | 86.4 | 87.4 | 88.2 | 89.2 | 85.6 |

The content of LiFSISF contained in the electrolytic solution increased in the order of battery cells 3B to 6B and the capacity retention rate after the trickle charge cycle increased in this order. When battery cells 3B and 4B are compared, the LiFSO₃ contents in the electrolytic solutions are equivalent; however, battery cell 4B containing a larger amount of LiFSISF shows a satisfactory result with respect to the capacity retention rate. From these, it is considered that the capacity retention rate after the trickle charge cycle performed at a high temperature was improved by adding LiFSISF in the electrolytic solution.

LiPF₆ and electrolyte compositions of Examples 3B to 6B and Comparative Example 2B were individually dissolved in a solvent (EC:MEC = 3:7 (volume ratio)) to prepare electrolytic solutions 7B to 10B and C4B having a volume of 10 mL. The concentration of LiPF₆ in electrolytic solutions 7B to 10B and C4B was 0.95 M and the electrolyte compositions of Examples 3B to 6B and Comparative Example 2B were added to the respective electrolytic solutions such that the concentration of FSI anion became 0.05 M. Note that, as LiPF₆, a commercially available product was used.

The actual weighed values of the electrolyte compositions and the contents of LiFSISF and LiFSO₃ in the electrolytic solutions calculated from the analysis results of Table 7 are shown in Table 11.

**[Table 11]**

| | Electrolytic solution 7B | Electrolytic solution 8B | Electrolytic solution 9B | Electrolytic solution 10B | Electrolytic solution C4B |
|---|---|---|---|---|---|
| Electrolyte composition/g | 0.094 | 0.094 | 0.095 | 0.095 | 0.094 |
| LiFSISF/mass ppm | 1 | 2 | 85 | 183 | N.D. |
| LiSO₃/mass ppm | 19 | 19 | 14 | 112 | N.D. |

Using electrolytic solutions 7B to 10B and C4B, battery cells were assembled in the same manner as in battery cell 3B. These battery cells were subjected to aging in aging condition 2 as mentioned above to accomplish battery cells 7B to 10B and C4B. Battery cells 7B to 10B and C4B were subjected to a test carried out in the conditions of Cycle characteristics 2 at 45°C to obtain capacity retention rates. The results are shown in Table 12.

**[Table 12]**

| | Battery cell 7B | Battery cell 8B | Battery cell 9B | Battery cell 10B | Battery cell C4B |
|---|---|---|---|---|---|
| Capacity retention rate/% | 72.5 | 72.7 | 73.4 | 74.2 | 72.4 |

The content of LiFSISF (contained) in the electrolytic solution increased in the order of battery cells 7B to 10B and the capacity retention rate after the trickle charge cycle increased in this order. When battery cells 7B and 8B are compared, the LiFSO₃ contents in the electrolytic solutions are equivalent; however, battery cell 8B containing a larger amount of LiFSISF shows a satisfactory result with respect to the capacity retention rate. From these, it is considered that a capacity retention rate after the cycle test performed at a high temperature was improved by adding LiFSISF in the electrolytic solution.

As described in the above, the high temperature cycle characteristics of a battery cell was improved by using an electrolyte composition containing a predetermined amount of FSISF.

As the reason thereof, which is not clearly known, the present inventors consider as follows: FSISF anions are decomposed and adsorbed to form a film onto positive and negative electrode surfaces, thereby suppressing oxidative-reductive decomposition of the electrolytic solution in a high temperature condition, with the result that production of a decomposition product and deposition thereof on the negative electrode are suppressed.

## Claims

1. A compound represented by the following formula (1): 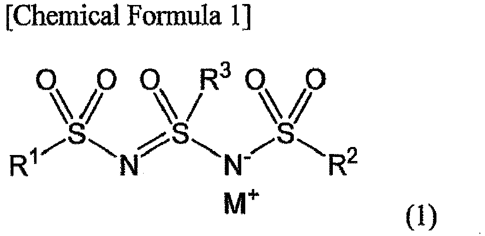 wherein R¹, R² and R³ each independently represent fluorine, an alkyl group having 1 to 6 carbon atoms or a fluoroalkyl group having 1 to 6 carbon atoms; and M⁺ represents an alkali metal ion.

2. The compound according to Claim 1, wherein each R¹, R² and R³ in the formula (1) is a fluorine atom and M⁺ is Li⁺.

3. An electrolyte composition comprising a compound represented by the following formula (1) and a compound represented by the following formula (2), wherein the content of the compound of the formula (1) in the electrolyte composition is 1 mass ppm to 200000 mass ppm; and the content of the compound of the formula (2) is 100000 mass ppm or more, 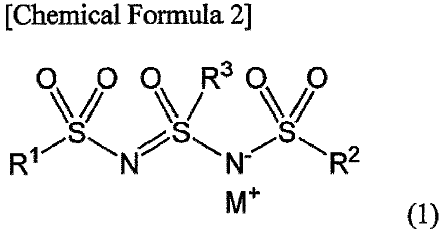 wherein R¹, R² and R³ each independently represent fluorine, an alkyl group having 1 to 6 carbon atoms or a fluoroalkyl group having 1 to 6 carbon atoms; and M⁺ represents an alkali metal ion,
(XSO₂)(XSO₂)NM ... (2)
wherein X represents a fluorine atom, an alkyl group having 1 to 6 carbon atoms or a fluoroalkyl group having 1 to 6 carbon atoms; a plurality of X may be the same or different; and M represents an alkali metal.

4. The electrolyte composition according to Claim 3, wherein each R¹, R² and R³ in the formula (1) is a fluorine atom, M⁺ is Li⁺ and each X in the formula (2) is a fluorine atom.

5. The electrolyte composition according to Claim 3 or 4, further comprising 100 mass ppm to 100000 mass ppm of MFSO₃ (M represents an alkali metal).

6. A method for producing a compound of the following formula (1), comprising a step of heating a solid-state compound of the following formula (2) at 150°C or more, 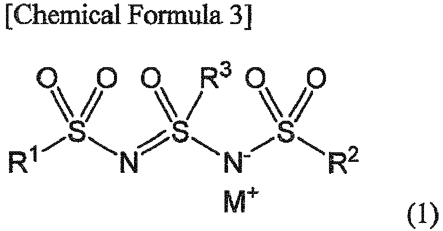 wherein R¹, R² and R³ each independently represent fluorine, an alkyl group having 1 to 6 carbon atoms or a fluoroalkyl group having 1 to 6 carbon atoms; and M⁺ represents an alkali metal ion,
(XSO₂)(XSO₂)NM ... (2)
wherein X represents a fluorine atom, an alkyl group having 1 to 6 carbon atoms or a fluoroalkyl group having 1 to 6 carbon atoms; a plurality of X may be the same or different; and M represents an alkali metal.

7. A solution containing 0.1 mass ppm to 180000 mass ppm of a compound represented by the following formula (4): 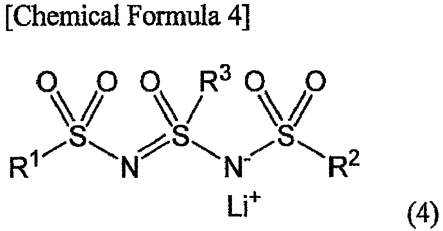 wherein R¹, R², R³ each independently represent fluorine or a fluoroalkyl group having 1 to 6 carbon atoms.

8. The solution according to Claim 7, further containing 0.1 mass ppm to 10000 mass ppm of L₁FSO₃.

9. The solution according to Claim 7 or 8, further containing at least one compound selected from the group consisting of LiPF₆, LiBF₄ and LiN(SO₂CₙF₂ₙ₊₁)₂ wherein n = an integer of 0 to 6.

10. The solution according to any one of Claims 7 to 9, further containing 1 mass ppm to 90 mass% of lithium bis(fluorosulfonyl)imide.

11. The solution according to any one of Claims 7 to 10, wherein each R¹, R² and R³ is a fluorine atom.

12. An electrolytic solution containing the solution according to any one of Claims 7 to 11.

13. A lithium ion battery comprising the electrolytic solution according to Claim 12.
